# FASCICULE DE BREVET EUROPEEN

(11) **EP 1 748 771 B1**
(45) Date de publication et mention de la délivrance du brevet: **30.04.2014**
(21) Numéro de dépôt: 05762397.7
(22) Date de dépôt: 22.04.2005
(51) Int. Cl.: A61K 31/22, A61K 31/122, A61K 31/045, A61K 31/015, A61P 17/00

(54) **COMPOSITION POUR PREVENIR OU TRAITER LA DEGENERESCENCE CELLULAIRE EN UTILISANT AU MOINS UNE MOLECULE CAPABLE DE MANTENIR LA REVERSIBILITE DE L'EXPRESSION DES MOLECULES D'ADHERENCE ET LA POLYMERISATION DES FIBRES D'ACTINE DE L'ENDOTHELIUM VASCULAIRE**
ZUSAMMENSETZUNG ZUR BEHANDLUNG ODER VORBEUGUNG VON ZELLDEGENERATION UNTER VERWENDUNG VON MINDESTENS EINEM MOLEKÜL ZUR ERHALTUNG DER REVERSIBILITÄT VON ADHÄSIONSMOLEKÜLEXPRESSION UND POLYMERISATION VON GEFÄSSENDOTHELIALEN ACTINFASERN
COMPOSITION FOR TREATING OR PREVENTING CELL DEGENERATION USING AT LEAST ONE MOLECULE CAPABLE OF MAINTAINING ADHESION MOLECULE EXPRESSION REVERSIBILITY AND VASCULAR ENDOTHELIUM ACTIN FIBRE POLYMERISATION

(30) Priorité: 23.04.2004 FR 0404344
(43) Date de publication de la demande: 07.02.2007
(73) Titulaire: AISA THERAPEUTICS, 91058 Evry Cedex (FR)
(72) Inventeur: D'ALESSIO, Patrizia, 75007 Paris (FR)
(74) Mandataire: Jacobson, Claude
(86) Numéro de dépôt international: PCT/FR2005/001008
(87) Numéro de publication internationale: WO 2005/105074

(56) Documents cités:
- WO-A-01/78706
- WO-A-02/13840
- WO-A1-99/35116
- DE-A1- 19 644 422
- DE-A1- 19 915 102
- FR-A- 2 671 721
- US-A- 5 599 546
- US-A1- 2003 104 089
- US-A1- 2003 199 592
- US-B1- 6 177 472
- PATENT ABSTRACTS OF JAPAN vol. 1999, no. 05, 31 mai 1999 (1999-05-31) & JP 11 035455 A (NOEVIR CO LTD), 9 février 1999 (1999-02-09)
- STOHS S J: "THE ROLE OF FREE RADICALS IN TOXICITY AND DISEASE" JOURNAL OF BASIC AND CLINICAL PHYSIOLOGY AND PHARMACOLOGY, FREUND PUBLISHING HOUSE LTD., LONDON, GB, vol. 6, no. 3/4, 1995, pages 205-228, XP002040436 ISSN: 0792-6855
- DATABASE WPI Section Ch, Week 200416 Derwent Publications Ltd., London, GB; Class B04, AN 2004-160857 XP002331736 & JP 2004 002237 A (AMANO JITSUGYO KK) 8 janvier 2004 (2004-01-08)
- DATABASE WPI Week 200222, Derwent Publications Ltd., London, GB; AN 2002-169193 & KR 2001 0 086 473 A (KOREA ADV INST SCI & TECHNOLOGY) 12 Septembre 2001
- DATABASE WPI Week 199301, Derwent Publications Ltd., London, GB; AN 1993-005497 & JP 4 334 319 A (SEKISUI CHEM IND CO LTD) 20 Novembre 1992

## Description

La présente invention concerne l'étude de la réversion de la sénescence des tissus par la médiation des cellules endothéliales vasculaires. Plus particulièrement, la présente invention concerne les effets de composés d'origine végétale de type terpène sur l'endothélium vasculaire lors d'épisodes inflammatoires. Ces composés sont capables d'inhiber l'expression des molécules d'adhérence endothéliales et la polymérisation des fibres d'actine endothéliales lors d'épisodes inflammatoires. Cette inhibition protège les tissus sous-jacents de l'endothélium des effets toxiques de l'inflammation qui précipitent leur dégénérescence.

En effet, l'inflammation répétée ou chronique au niveau de l'endothélium vasculaire induit sa sénescence accélérée, ainsi que la dégénérescence des tissus soumis au processus inflammatoire.

La sénescence d'une cellule est un phénomène qui entraîne une augmentation du volume de la cellule, une diminution ou une perte de la capacité normale de la cellule à se diviser, une diminution des aptitudes régénératrices et métaboliques, et une augmentation de l'activité des enzymes cellulaires de dégradation. Toutes ces caractéristiques définissent un phénotype sénescent.

Ce phénotype sénescent a été observé sur des cellules jeunes soumises à des stress répétés dus à des réactions inflammatoires.

La phase initiale de l'inflammation se déroule à l'interface sang/tissu et consiste dans le recrutement des cellules immunocompétentes, en particulier les leucocytes activés. L'inflammation consiste à cibler et à détruire les microorganismes infectieux et autres agents pathogènes. Les cellules immunocompétentes susceptibles de lutter contre ces microorganismes circulent dans le sang et doivent donc se rendre dans les tissus pour y exercer leur action ; pour cela, elles doivent franchir la barrière de la paroi vasculaire. Elles y parviennent en étant recrutées par les cellules endothéliales vasculaires qui tapissent les vaisseaux, et qui vont les transférer au niveau des tissus sous-jacents. Les cellules endothéliales vasculaires sont activées par des signaux spécifiques issus du tissu infecté ou blessé, ou directement par les cellules immunocompétentes et cette activation a pour effet d'augmenter sensiblement leur capacité de recrutement. On peut mesurer l'état d'activation des cellules endothéliales vasculaires par l'apparition de marqueurs spécifiques. En particulier, le niveau d'expression de molécules d'adhérence telles qu'ICAM-1 (INTER CELLULAR ADHESION MOLECULE-1) permet d'évaluer l'état d'activité des cellules endothéliales vasculaires aboutissant au recrutement des cellules immunocompétentes.

Une autre conséquence du stress inflammatoire est le remaniement du cytosquelette d'actine des cellules endothéliales vasculaires activées. Le stress inflammatoire entraîne la formation de fibres d'actine polymérisées (dites « de stress ») que l'on peut mettre en évidence par le marquage à la phalloïdine couplée à la rhodamine.

Dans une situation normale, dans une culture cellulaire primaire de cellules endothéliales vasculaires, l'activation correspondant à un état inflammatoire de la cellule est réversible. En situation de sénescence, la cellule perd progressivement cette réversibilité et exprime alors de manière permanente des molécules d'adhérence, même en l'absence de stimulation extérieure. Cette perte de réversibilité à deux conséquences : d'une part, le recrutement constant des cellules immunocompétentes est une source de stress inflammatoire supplémentaire pour les tissus ; et d'autre part, la cellule endothéliale vasculaire possède de ce fait une disponibilité accrue pour le recrutement de métastases de tumeurs, qui utilisent les mêmes molécules d'adhérence pour envahir les tissus.
Le document JP11-035455 divulgue l'effet du géraniol sur la prévention du vieillissement de la peau, traitement de blessures.
DE19915102 et WO01178706 montrent l'effet du limonène sur le traitement du vieillissement de la peau et celui lié à l'exposition aux rayons UV.
WO02113840 se rapporte à l'utilisation du limonène pour le traitement de l'ostéoporose comme conséquence du vieillissement.
Stohs S. J. (Journal of Basic and Clinical Physiology and Pharmacology (1995), 6 (3/4), 205 - 228) décrit le rôle du limonène comme capteur de radicaux libres.
US5599546 mentionne un masque cosmétique comprenant l'huile de limonène et son utilisation pour le traitement du vieillissement de la peau.
FR2671721 divulgue un produit pour la re-pigmentation des cheveux blancs suite au vieillissement comprenant du limonène et l'extrait de Carum carvi.

L'abrégé Derwent **WPI 2004-160857** montre l'utilisation du géraniol pour le traitement du vieillissement.

Un autre agrégé Dennrent, **WPI 2002-169193** se rapporte à utilisation du limonène et du géraniol pour le traitement de l'asthme, psoriasis, arthrite, inflammation des intestins, arthrite rhumatoïde, rhinite allergique par inhibition de la production de leucotriènes. DE19644422 décrit l'utilisation du limonène, du géraniol et d'autres terpènes pour le traitement de maladies auto-immunes par inhibition de l'activation des lymphocytes T. US2003/104089 divulgue un extrait comprenant du limonène et son utilisation pour la prévention des métastases de tumeurs par inhibition de l'expression de cycline D1. WO99/35116 décrit l'utilisation d'une composition comprenant des diterpènes de formule (I) et éventuellement du limonène pour le traitement de conditions inflammatoires, auto-immunes et allergiques par inhibition de réactions d'hypersensibilité liées aux immunoglobulines ou aux antigènes.

L'abrégé Derwent **WPI 1993**-**005497** montre l'utilisation de l'acétate de géranyl pour le traitement de dermatite inflammatoire provoquée par des allergies par inhibition de réactions d'hypersensibilité.

US20031199592 se rapporte à l'utilisation du limonène pour le traitement de la maladie de Crohn, la sénescence des cellules endothéliales ou d'autres mécanismes n'étant pas mentionnés.

US6177472 divulgue l'utilisation de l'isomenthone pour le traitement de la maladie d'Alzheimer par inhibition de la libération d'acide gras pour réguler l'assemblage de la protéine Tau.

En conséquence, la présente invention a pour objet l'utilisation d'une molécule choisie dans le groupe comprenant l'acétate de géranyl, le géraniol, l'isomenthone, le limonène, ou le mélange d'au moins deux de celles-ci pour la préparation d'un médicament utile pour prévenir ou traiter la dégénérescence des tissus associée à la sénescence des cellules endothéliales vasculaires induite par des épisodes inflammatoires répétés.

Ces molécules ont en commun leur capacité à inhiber l'activation irréversible des cellules endothéliales vasculaires et de maintenir ces cellules dans un état d'activation fonctionnelle dans lequel elles sont capables de répondre à une stimulation environnementale et de revenir ensuite à leur état de base. Ces molécules sont capables d'inverser le phénotype sénescent de la cellule endothéliale vasculaire alors qu'il est déjà constaté et elles sont donc cytoprotectrices, car elles protègent les cellules des tissus sous-jacents des effecteurs inflammatoires sécrétés par les cellules immunocompétentes activées, empêchant ainsi leur dégénérescence. L'utilisation selon l'invention fait intervenir au moins une molécule possédant cette capacité.

Ces molécules sont contenues ou issues d'huiles essentielles d'origine végétale. Avantageusement, elles sont isolées et purifiées ou sont préparées par synthèses chimiques.

L'acétate de géranyl est l'acétate de (E)-3,7-Dimethyl-2,6-octadienyl (CAS : 105-87-3). Il est présent, entre autres, dans les huiles essentielles de rose, de citronnelle, de nard indien (lemon grass), de géranium et de néroli.

Le géraniol est le (E)-3,7-dimethyl-2,6-octadien-1 ol (CAS : 106-24-1). Il est présent, entre autres, dans les huiles essentielles de rose, de néroli et de géranium.

L'isomenthone est le cis-2-Isopropyl-5-methylcyclohexanone (CAS : 491-07-6), présent entre autres, dans les huiles essentielles de la menthe et du géranium.

Le limonène est le (4R)-1-methyl-4-isopropenylcyclohex-1-ene (CAS : 5989-27-5). Il est présent, entre autres, dans les huiles essentielles du citron et du néroli.

Avantageusement, ces molécules sont utilisées selon l'invention dans une composition où elles sont présentes dans des quantités très faibles.

Suivant un premier mode de réalisation, l'invention s'intéresse la sénescence des cellules endothéliales vasculaires qui est induite par des épisodes inflammatoires répétés liés à la présence de cellules cancéreuses. En conséquence, l'invention se rapporte à la préparation d'un médicament pour traiter l'exportation des métastases favorisée par une surexpression des molécules d'adhérence par l'endothélium vasculaire et une polymérisation de l'actine endothéliale vasculaire, qui sont responsables du recrutement des cellules cancéreuses potentiellement métastatiques.

Suivant un deuxième mode de réalisation, l'invention s'intéresse à la sénescence des cellules endothéliales vasculaires et secondairement à la dégénérescence des tissus qui sont induits par des épisodes inflammatoires répétés provenant de l'exposition au soleil (UV), de la pollution, de micro-abrasion. En conséquence, l'invention se rapporte à la préparation d'un médicament pour traiter les affections dermatologiques ainsi que les allergies cutanées liées aux phénomènes ci-dessus qui entraînent une surexpression des molécules d'adhérence par l'endothélium vasculaire et une polymérisation de l'actine endothéliale vasculaire, associés aux épisodes inflammatoires qui induisent la dégénérescence des tissus dermiques.

Suivant un troisième mode de réalisation, l'invention s'intéresse à la sénescence des cellules endothéliales vasculaires et secondairement à la dégénérescence des tissus sous-jacents qui sont induits par des épisodes inflammatoires répétés associés aux maladies inflammatoires chroniques ou aux maladies auto-immunes. En conséquence, l'invention se rapporte à la préparation d'un médicament pour traiter les pathologies inflammatoires chroniques ou auto-immunes qui entraînent une surexpression des molécules d'adhérence par l'endothélium vasculaire et une polymérisation de l'actine endothéliale vasculaire, et qui induisent la dégénérescence des tissus sous-jacents.

Une pathologie inflammatoire chronique ou auto-immune est choisie dans le groupe comprenant la polyarthrite rhumatoïde, l'ostéoarthrite, les spondylarthrites, la goutte, l'arthrose et toutes les autres formes d'arthrites, l'hépatite chronique, la rectocolite hémorragique, la maladie de Crohn, les vasculites, la sclérose en plaques, le psoriasis, les lupus érythémateux et cutané ainsi que les sclérodermies.

Suivant un quatrième mode de réalisation, l'invention s'intéresse à la sénescence des cellules endothéliales vasculaires et secondairement à la dégénérescence des tissus qui sont induits par des épisodes inflammatoires répétés associés aux maladies dégénératives. En conséquence, l'invention se rapporte à la préparation d'un médicament pour traiter les maladies dégénératives cérébrales qui sont associées à une surexpression des molécules d'adhérence par l'endothélium vasculaire et une polymérisation de l'actine endothéliale vasculaire qui induisent la dégénérescence des tissus cérébraux.

Une pathologie dégénérative cérébrale est choisie dans le groupe comprenant la maladie d'Alzheimer, les démences séniles vasculaires ou mixtes, la maladie de Parkinson, la maladie de Huntington.

Avantageusement, ce médicament est administrable par voie topique, orale, entérale, parentérale ou inhalation.

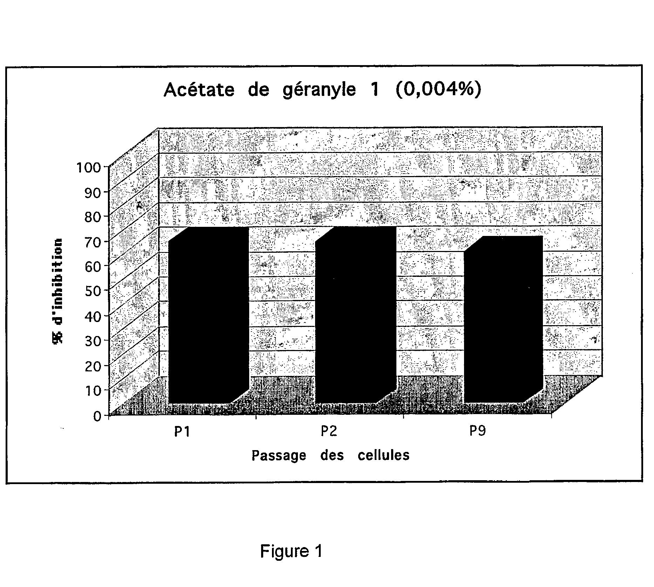

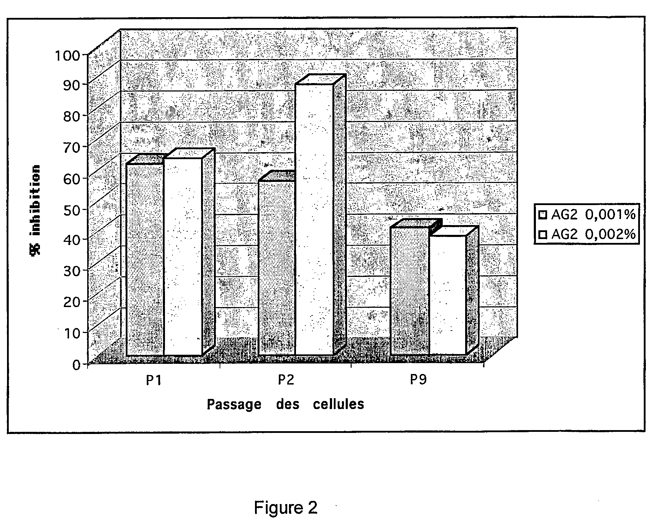

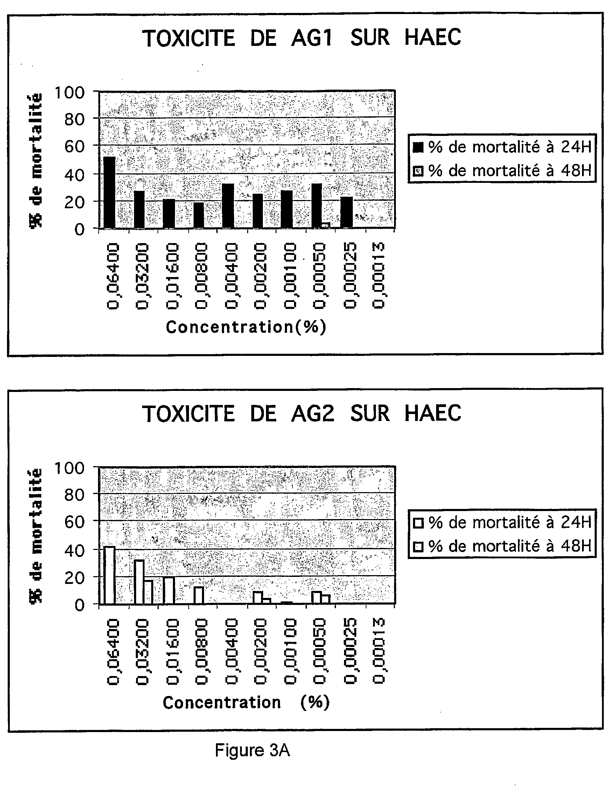

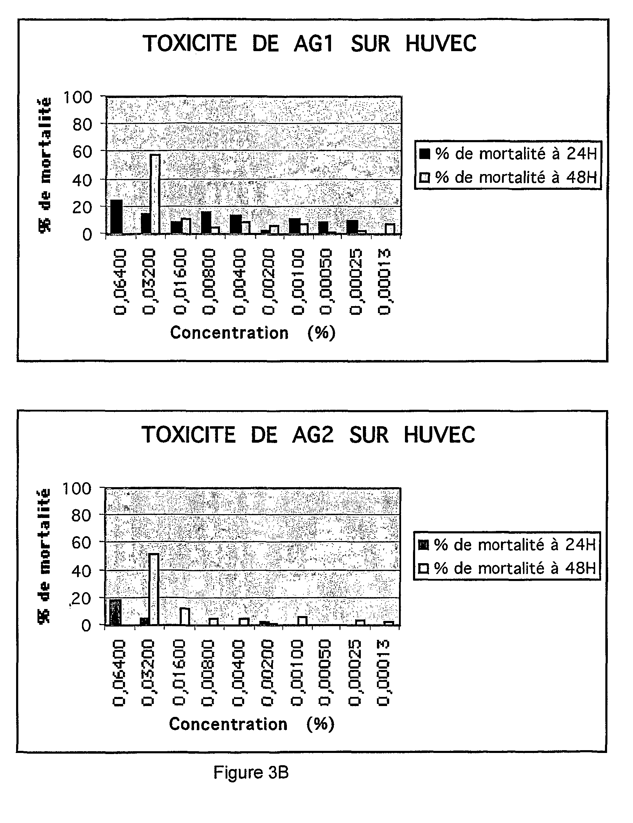

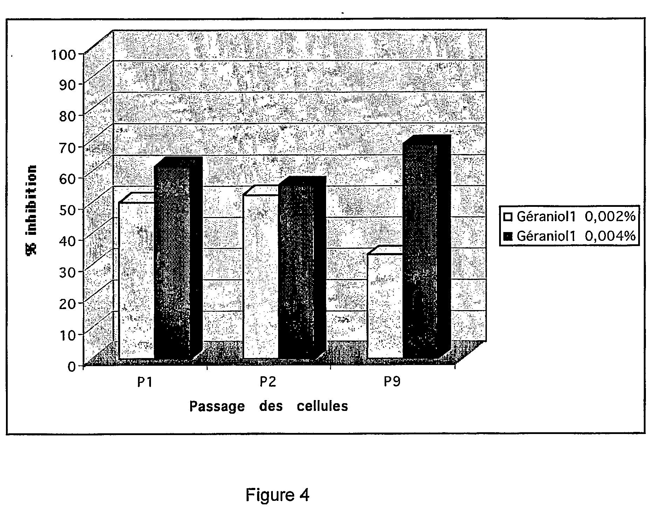

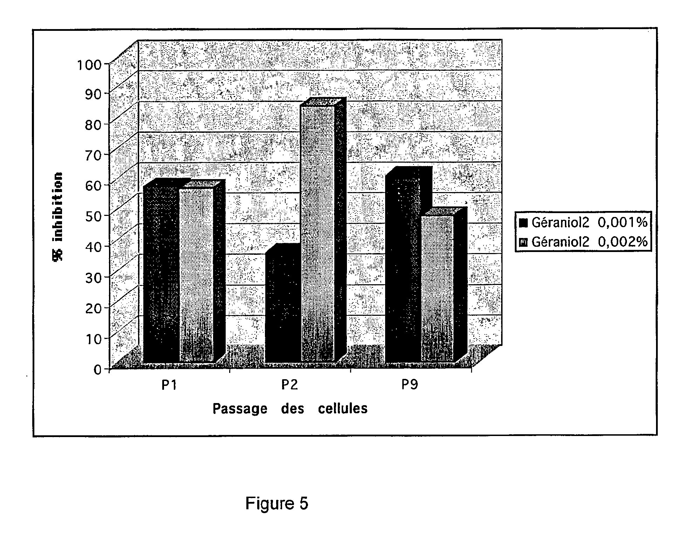

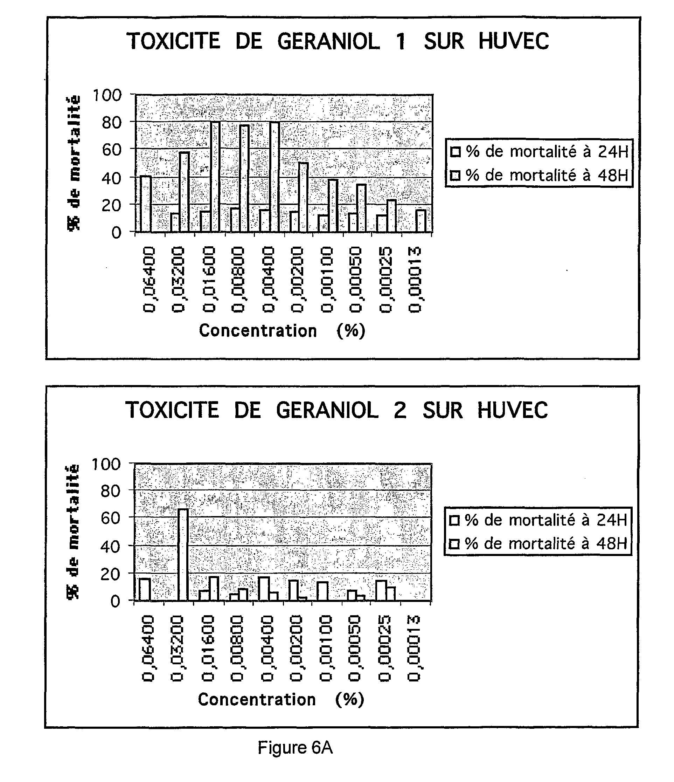

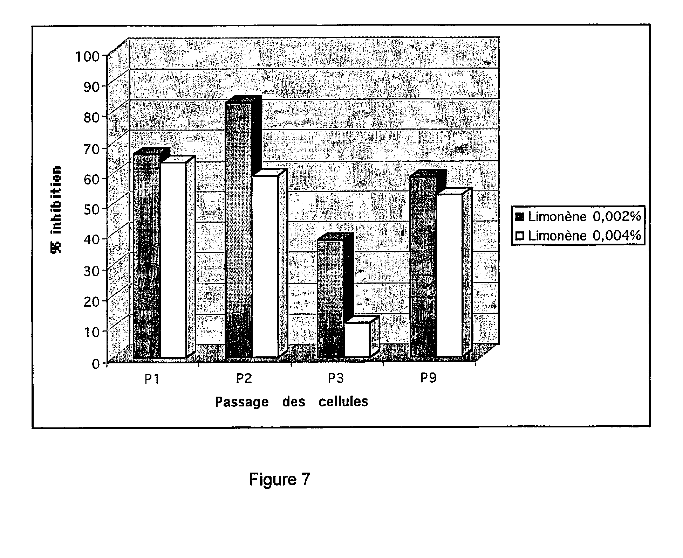

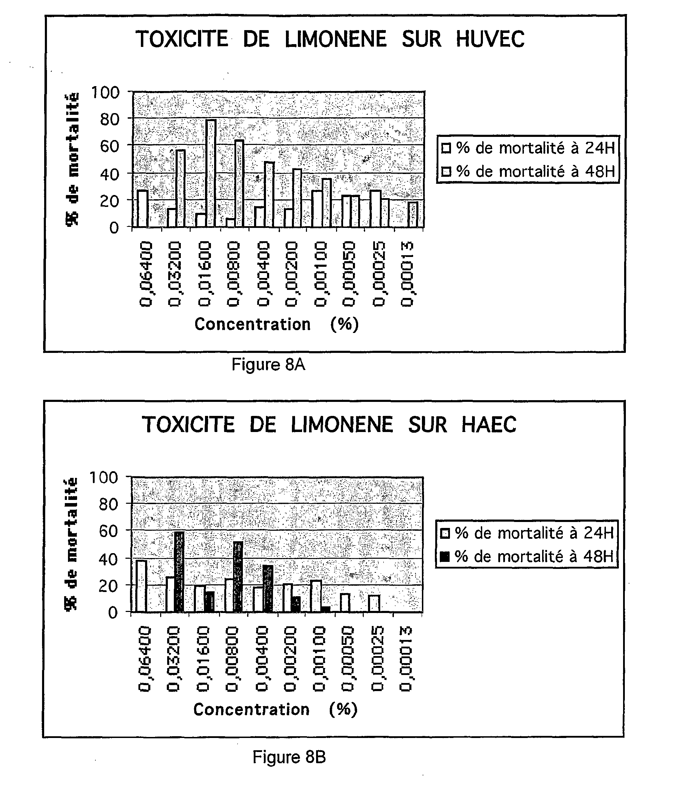

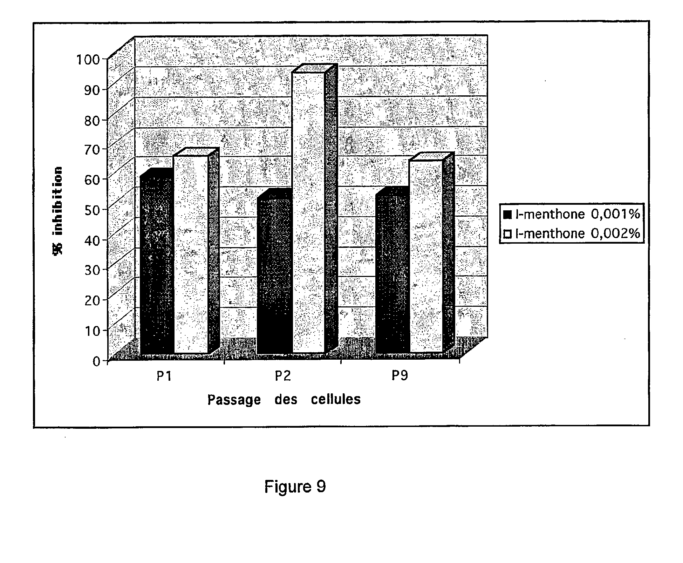

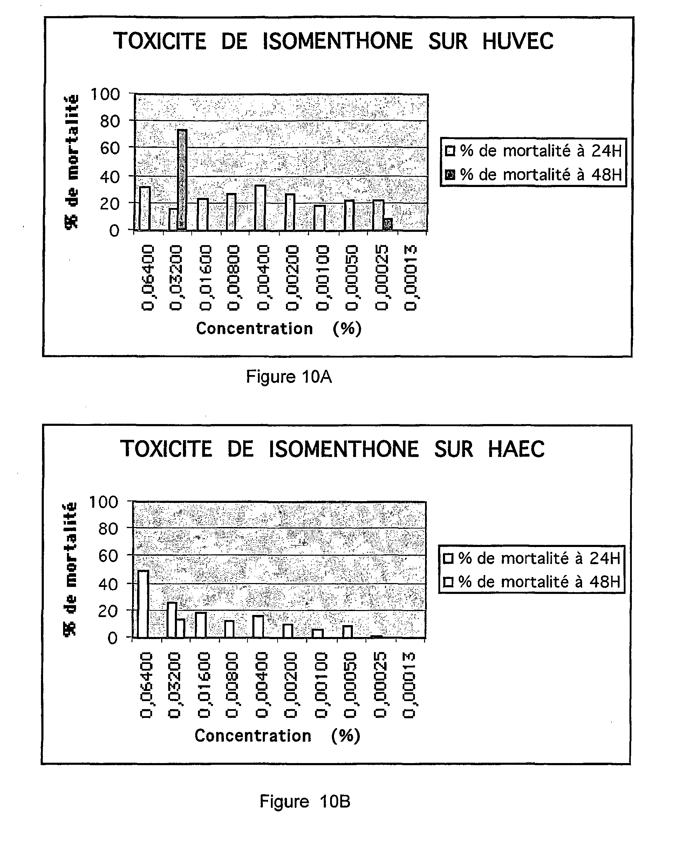

D'autres avantages et caractéristiques de l'invention apparaîtront dans les exemples qui suivent concernant l'inhibition des molécules d'adhérence et de la polymérisation de l'actine des cellules endothéliales vasculaires sous l'action de l'acétate de géranyl, du géraniol, de l'isomenthone, du limonène. Il sera fait référence dans ces exemples aux dessins en annexe dans lesquels :
1/ Acétate de géranyl Figures 1, 2, 3A, 3B
   - la figure 1 montre le pourcentage d'inhibition de l'expression d'ICAM-1 des cellules HUVEC (Human Umbilical Vein Endothelial Cells) à différentes étapes de réplication (P1, P2 correspondant à des populations de cellules jeunes, peu répliquées, et P9 à des cellules ayant atteint la limite de leurs taux de réplication, donc sénescentes), par l'acétate de géranyle à 0,004 % suite à une stimulation par le TNF-α à la concentration de 10ng/mL pendant 24 heures.
   - la figure 2 montre le pourcentage d'inhibition de l'expression d'ICAM-1 des cellules HUVEC à différentes étapes de réplication par l'acétate de géranyle à 0,001 % ou 0,002 % suite à une stimulation par le TNF-α à la concentration de 10ng/mL pendant 24 heures.
   - la figure 3A montre la toxicité de l'acétate de géranyle, testé à différentes concentrations sur des cellules HAEC (HUMAN AORTIC ENDOTHELIAL CELLS), la toxicité étant exprimée en % de mortalité des cellules endothéliales HAEC à 24H et à 48H.
   - la figure 3B montre la toxicité de l'acétate de géranyle, testé à différentes concentrations sur des cellules HUVEC, la toxicité étant exprimée en % de mortalité des cellules endothéliales HUVEC à 24H et à 48H.
2/ Géraniol, Figures 4, 5, 6A 6B
   - la figure 4 montre le pourcentage d'inhibition de l'expression d'ICAM-1 des cellules HUVEC à différentes étapes de réplication par le géraniol à 0,002% et 0,004% suite à une stimulation par le TNF-α à la concentration de 10ng/mL pendant 24 heures.
   - la figure 5 montre le pourcentage d'inhibition de l'expression d'ICAM-1 des cellules HUVEC à différentes étapes de réplication par le géraniol à 0,001% et 0,002% suite à une stimulation par le TNF-α à la concentration de 10ng/mL pendant 24 heures.
   - la figure 6A montre la toxicité du géraniol, testé à différentes concentrations sur des cellules HUVEC, la toxicité étant exprimée en % de mortalité des cellules endothéliales HUVEC à 24H et à 48H.

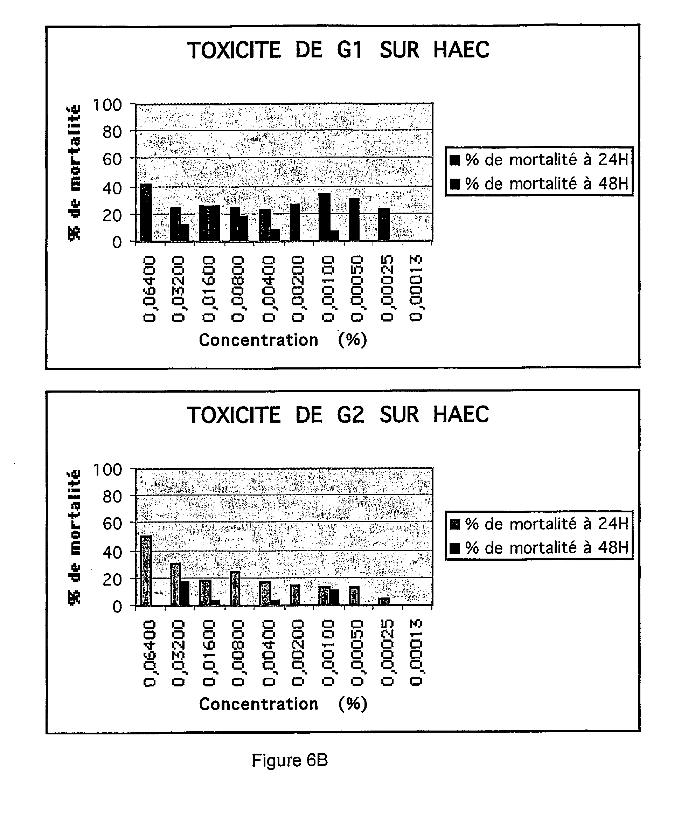

   - la figure 6B montre la toxicité du géraniol, testé à différentes concentrations sur des cellules HAEC, la toxicité étant exprimée en % de mortalité des cellules endothéliales HAEC à 24H et à 48H.
3/ Limonène, Figures 7, 8A, 8B
   - la figure 7 montre le pourcentage d'inhibition de l'expression d'ICAM-1 des cellules HUVEC à différentes étapes de réplication par le limonène à 0,002% et 0,004% suite à une stimulation par le TNF-α à la concentration de 10ng/mL pendant 24 heures.
   - la figure 8A montre la toxicité du limonène, testé à différentes concentrations sur des cellules HUVEC, la toxicité étant exprimée en % de mortalité des cellules endothéliales HUVEC à 24H et à 48H.
   - la figure 8B montre la toxicité du limonène, testé à différentes concentrations sur des cellules HAEC, la toxicité étant exprimée en % de mortalité des cellules endothéliales HAEC à 24H et à 48H.
4/iso-menthone, figures 9, 10A, 10B
   - la figure 9 montre le pourcentage d'inhibition de l'expression d'ICAM-1 des cellules HUVEC à différentes étapes de réplication par l'iso-menthone à 0,001% et 0,002% suite à une stimulation par le TNF-α à la concentration de 10ng/mL pendant 24 heures.
   - la figure 10A montre la toxicité de l'iso-menthone, testé à différentes concentrations sur des cellules HUVEC, la toxicité étant exprimée en % de mortalité des cellules endothéliales HUVEC à 24H et à 48H.
   - la figure 10B montre la toxicité de l'iso-menthone, testé à différentes concentrations sur des cellules HAEC, la toxicité étant exprimée en % de mortalité des cellules endothéliales HAEC à 24H et à 48H.
   - Les figures 11 à 25 représentent l'étude de la toxicité *in vivo* des molécules « NAT » et « SYN » formes naturelle et synthétique de l'acétate de géranyl, molécule dénommée « AISA 5201 » et choisie quant à sa représentativité de la classe AISA, dans un modèle murin sur les paramètres de la numération sanguine, à des concentrations de 5, 10, 25 et 50 microgr / souris, correspondantes à des concentration non toxiques *in vitro,* permettant d'observer l'effet biologique voulu, i.e. l'inhibition de l'adhérence endothéliale, ainsi que de la polymérisation de l'actine endothéliale.

Exemple 1 : Les tests ont été réalisés sur des cellules endothéliales de différentes origines (de la veine de cordon (HUVEC), de la microcirculation de la peau (HMVEC), et de l'aorte (HAEC)), qui ont été soumises à différents stress (H₂O₂, Tumor Necrosis Factor alpha (TNF-α) pendant 2 heures, 6 heures, 24 heures et 48 heures) induisant l'activation de ces cellules. L'activation est mesurée en analysant l'expression des marqueurs tels qu'ICAM-I, VCAM-I (VASCULAR ADHESION MOLECULE-1), par les trois techniques suivantes : tests ELISA, cytométrie de flux, immuno-microscopie confocale à fluorescence.

Exemple 2 : Dosage immunoenzymatique ELISA : après stimulation par le TNF-α, les cellules sont lavées au PBS puis fixées au formaldéhyde 2% pendant 10 minutes. Les sites de fixation non spécifiques sont ensuite saturés par du lait écrémé à 5 %. Les cellules sont ensuite incubées avec l'anticorps primaire anti-ICAM-I (R&D) à 0,02 µg/ml pendant deux heures à température ambiante, puis lavées cinq fois au PBS. Elles sont ensuite incubées avec le complexe de révélation, anti-IgG de souris lié à la phosphatase alkaline (Chemicon, Euromedex) pendant une heure à température ambiante, puis lavées cinq fois avec du PBS. La révélation colorimétrique est réalisée par le substrat de la phosphatase alcaline, le para-nitrophénylphosphate à 1 mg/ml. La réaction est arrêtée après 20 mn d'incubation par NaOH 1M. La lecture se fait au lecteur de plaques à 405 nm.

La cytométrie de flux mesure aussi l'expression des protéines marquées par un anticorps spécifique, l'anticorps portant cette fois-ci un groupement fluorescent. L'Elisa permet une mesure globale de l'expression des molécules d'adhérence, la cytométrie de flux permettant éventuellement de discriminer des sous-populations présentant un profil d'expression variable. Les cellules sont lavées au PBS contenant entre 0.5-2% d'albumine de sérum bovin, puis resuspendues dans une solution de PBS-BSA contenant une concentration optimale d'anticorps anti-molécules d'adhérence couplés à un fluorophore. Après lavage, la suspension de cellules est soumise au cytomètre de flux qui mesure la fluorescence spécifique associée à l'anticorps utilisé pour le marquage. Alternativement, les cellules peuvent subir une post-fixation (au PBS-BSA contenant 2% de para-formaldehyde) après les étapes de lavages finales en vue d'une mesure différée dans les sept jours suivants.

Exemple 3 : L'immuno-microscopie confocale à fluorescence est une méthode, de mise en évidence simultanée du réseau d'actine (par rhodamine couplée à la phalloïdine) et de molécules d'adhérence ICAM-1 ou VCAM-1 par marquage avec un anticorps couplé au FITC dans des cellules endothéliales vasculaires. La sénescence réplicative est associée à des modifications des interactions entre molécules d'adhérence et cytosquelette d'actine des cellules endothéliales vasculaires, ce que l'implémentation de cette mesure permet d'évaluer. Les cellules sont cultivées sur des lamelles en verre dans des puits de culture. Après 2, 6, ou 24 heures de stimulation par le TNF-α, les cellules sont lavées au PBS, puis fixées au para-formaldéhyde 2%. Les sites de fixation non spécifiques des anticorps sont ensuite saturés par du chlorure d'ammonium à 50mM, puis les cellules sont perméabilisées par la saponine 0,01%. Les lamelles avec les cellules fixées sont ensuite mises en contact avec l'anticorps primaire : anti-ICAM-1 couplé à FITC (DIACLONE) et à la Rhodamine (SIGMA P1951 PHALLOIDIN TRITC), pendant 1H30 puis lavées 2 fois au PBS. Dans le cas d'un marquage avec VCAM-1, les cellules sont mises en contact avec l'anticorps primaire : anti-VCAM-1 (IMMUNOTECH) pendant 1H30 puis lavées 2 fois au PBS. Elles sont alors incubées avec l'anticorps secondaire fluorescent : anti-souris Ig, « fluoresceine linked whole antibody » (AMERSHAM) et la rhodamine (SIGMA P1951 PHALLOIDIN TRITC), pendant 1H30 puis lavées 2 fois au PBS. Les lamelles sont ensuite incluses en glycérol sur des lames, puis observées en microscopie optique ou confocale.

Ces techniques permettent d'identifier, pour une cellule endothéliale vasculaire, l'entrée dans la phase de sénescence réplicative caractérisée par l'irréversibilité de la polymérisation de l'actine associée à la surexpression irréversible de molécules d'adhérence induite par le processus réplicatif physiologique et/ou le stress inflammatoire, mimé dans les tests par une stimulation au TNFα et/ou H₂O₂.

Les tests décrits dans les exemples 2 et 3 permettent de caractériser et.quantifier le potentiel d'une molécule à inhiber l'expression des molécules d'adhérence et la polymérisation de l'actine ce qui correspond à une inversion du phénotype de type sénescent de la cellule endothéliale vasculaire.

Exemple 4 : Etude préliminaire de toxicité sur une souche de souris sauvage, portant sur différentes concentrations de la molécule correspondant aux concentrations utilisées *in vitro.*

Suite aux résultats *in vitro* obtenus précédemment, il a été procédé à des études *in vivo* préliminaires sur la toxicité possible et les propriétés fonctionnelles de la molécule acétate de géranyl ici nommée « AISA-5201 ».

La toxicité aiguë de la molécule synthétique est connue. Chez le rat, la DL50 est de 6330 mg/kg, avec diverses altérations du comportement, allant de la somnolence au coma.

Pour l'étude de toxicité préliminaire, une souche de souris dénommée « C57BL6 » a été utilisée. Cette souche est connue pour sa DL50 pour le LipoPolySaccharide bactérien (LPS) à 600 microgr / souris (« Lipopolysaccharide-Induced Cytokine Cascade and Lethality in Ltα/TNFα-Deficient Mice » F. Amiot et al. 1997, Molecular Medicine vol 3 n12, 863-874), correspondant à une DL50 de 3000 mg/kg.

Deux protocoles ont été suivis, l'un avec la forme synthétique ici denommée « SYN » (catalogue SIGMA code produit 173495) et l'autre avec la forme naturelle non-filtrée (issue du traitement par HPLC à partir d'huiles essentielles de géranium) ici dénommée « NAT » de la dite molécule.

Sur la base des expériences de toxicité *in vitro,* dans un premier temps, les molécules « SYN » et « NAT » ont été testées sur les souris C57BL/6 à des concentrations de 10, 25 et 50 microgr / souris, ainsi qu'un lot de contrôle qui a reçu du PBS, en mode d'administration IP (intrapéritonéal) et rétro-orbitaire (IV). Chaque lot était constitué de 4 animaux, au total 16 animaux utilisés.

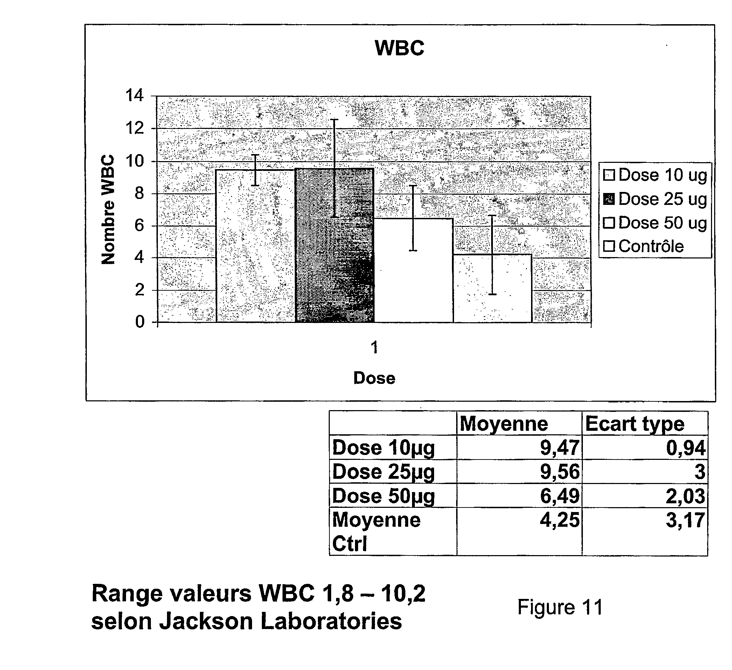

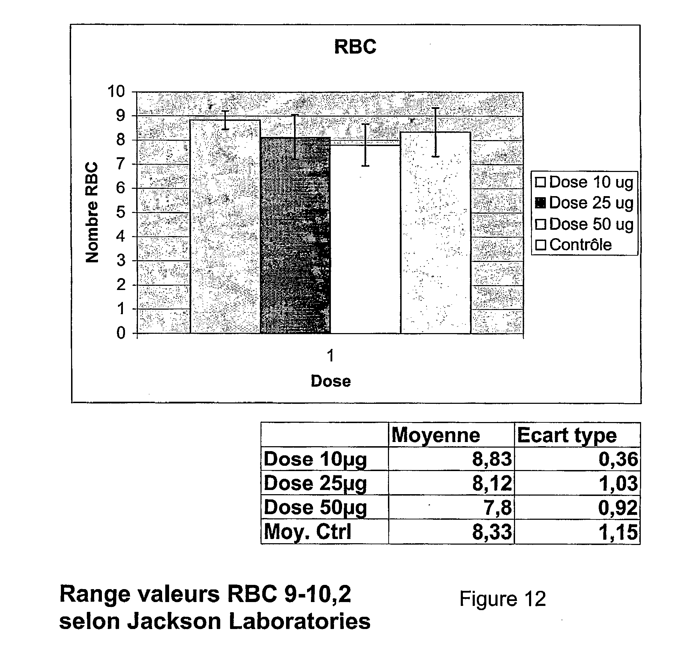

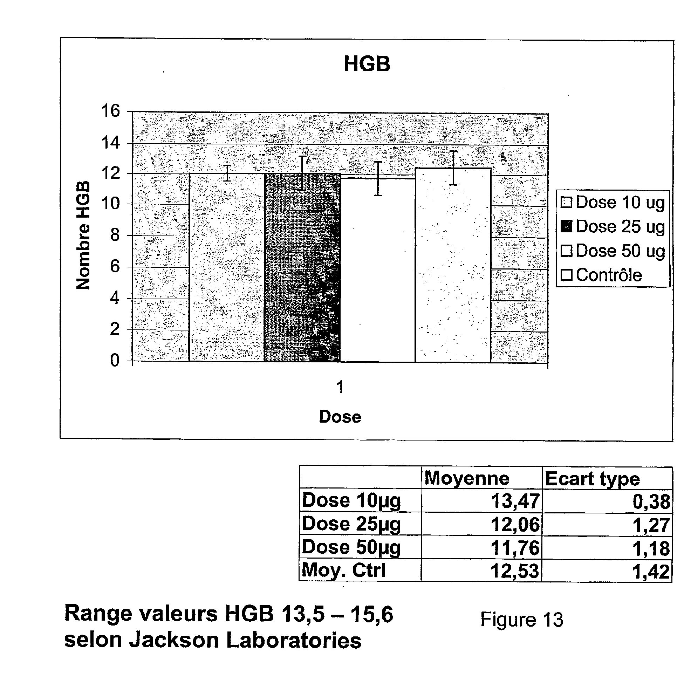

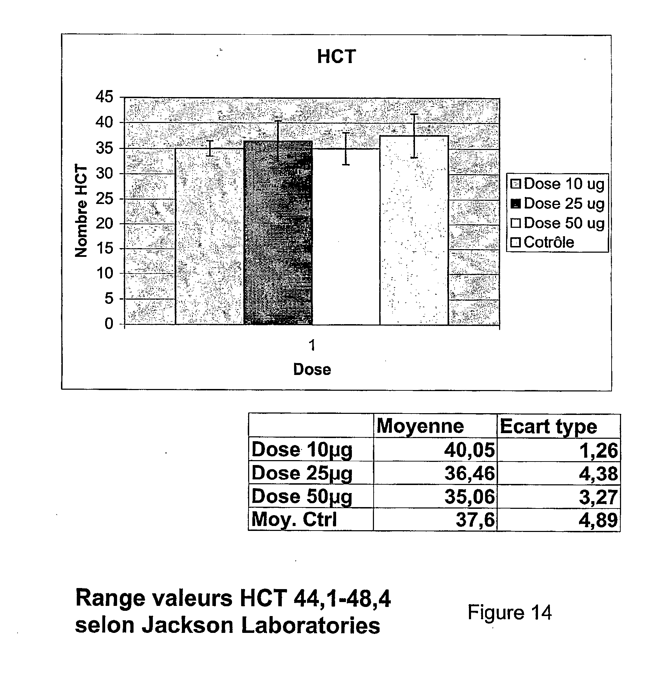

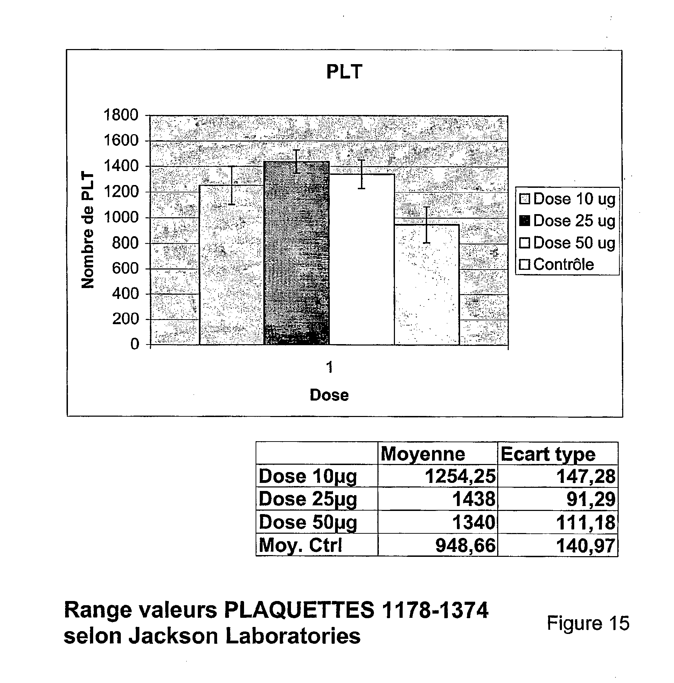

À l'issue de l'expérience, une numération sanguine a été effectuée sur la machine Cell Dyn Systems 3500/3700 Abbott Laboratories. Cette investigation été finalisée à mettre en évidence des éventuelles altérations fonctionnelles. Les résultats de cette expérience, toute forme « NAT » et « SYN » de la molécule confondue, nous a permis de mettre en évidence que la concentration de 50 microgr / souris était la plus appropriée, car les marqueurs de la lignée blanche (WBC, NEU, LYM, MONO) et des plaquettes (PLT) se maintenaient à des valeurs proches du lot du contrôle. Les marqueurs de la lignée rouge (RBC HGB et HTC) étaient par ailleurs stables à toutes les concentrations testées (figures 11 à 15).

Une deuxième expérience a été réalisée, en utilisant que la molécule de synthèse, à des concentrations de 5, 10 et 20 microgr / souris plus un lot de contrôle, pour à nouveau 4 animaux par lot, en mode IP uniquement.

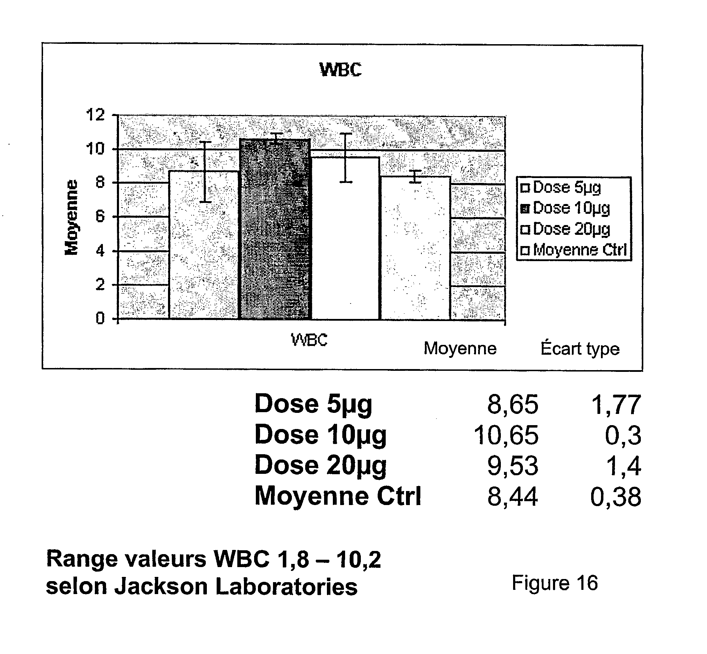

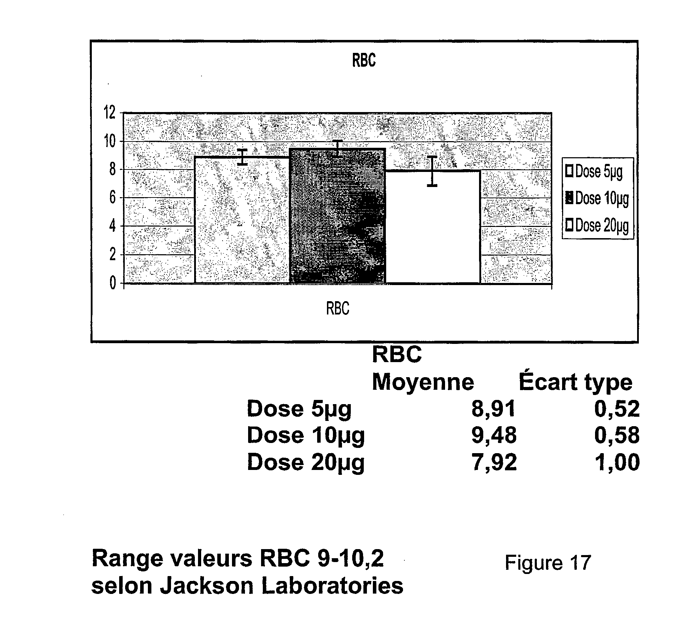

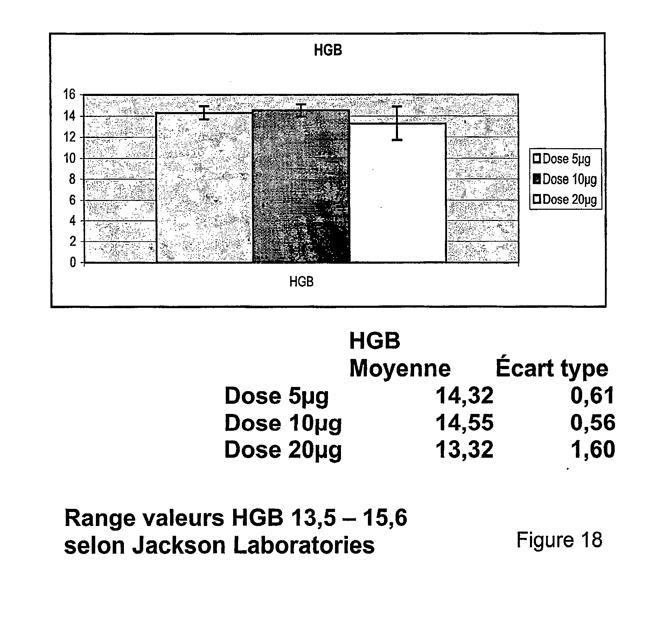

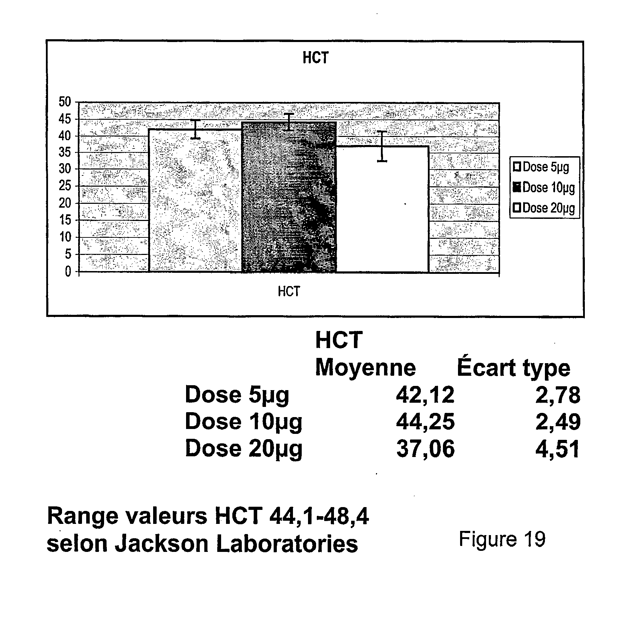

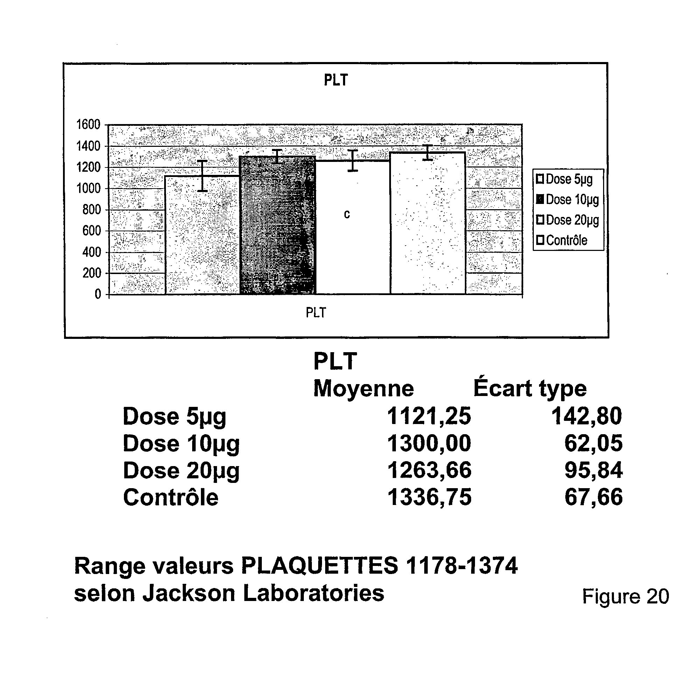

Puis, à l'issue de l'expérience, comme dans l'expérience précédente, une numération sanguine a été éffectuée. Les résultats (figures 16 à 20) montrent bien que la molécule « SYN » au concentration testées est stable, se maintenant aux valeurs du range standard.

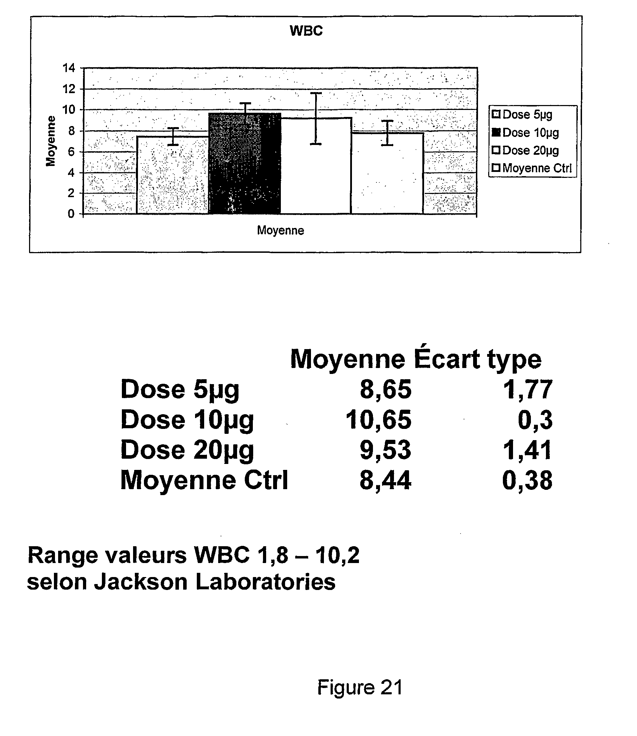

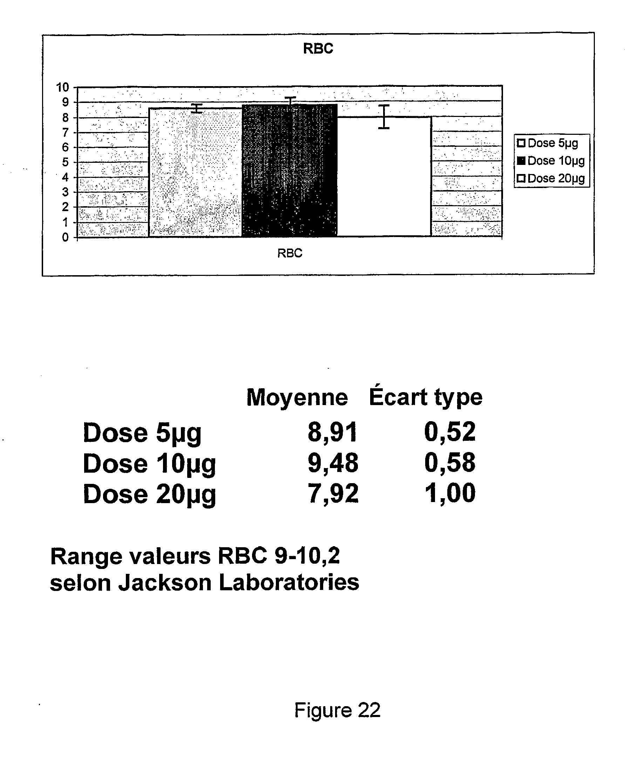

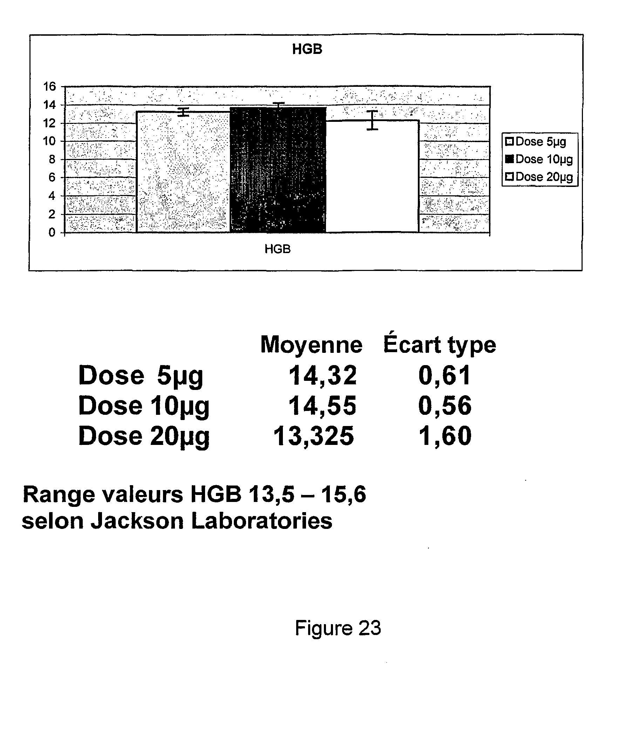

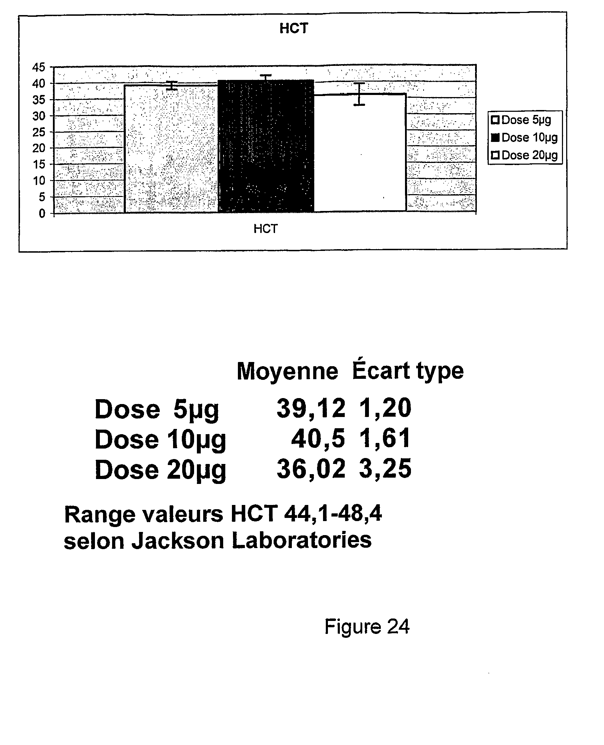

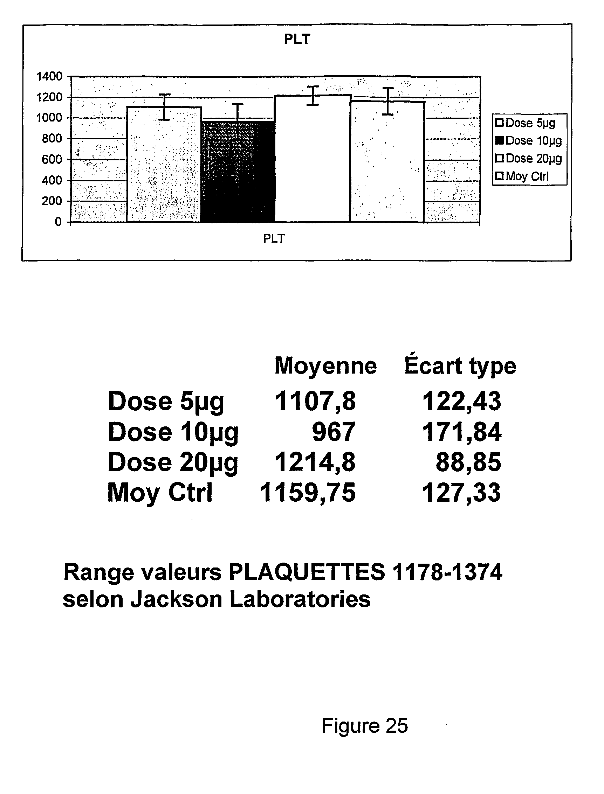

Une troisième expérience a été effectuée pour tester la molécule « NAT » aux mêmes concentrations que la »SYN », mais en augmentant le nombre d'animaux, c'est à dire 5 animaux par lot. Les résultats (figures 21 à 25) montrent bien que la molécule « NAT » au concentrations testées est stable, se maintenant aux valeurs du range standard.

Les molécules « NAT » et « SYN » formes naturelle et synthétique de l'acétate de géranyl, molécule dénommée « AISA 5201 » et choisie quant à sa représentativité de la classe AISA, sont comparables, quant à leur non-toxicité *in vivo* dans un modèle murin sur les paramètres de la numération sanguine, à des concentrations de 5, 10, 25 et 50 microgr / souris, correspondantes à des concentration non toxiques *in vitro*, permettant d'observer l'effet biologique voulu, i.e. l'inhibition de l'adhérence endothéliale, ainsi que de la polymérisation de l'actine endothéliale.

Le tableau 1 ci-après donne les valeurs standard de la numération sanguine dans la souris C57/BL6.

**Tableau 1**

| | | | | |
|---|---|---|---|---|
| | Dose 10 ug | Dose 25 ug | Dose 50 ug | Contrôle |
| WBC range | 8,13 -11,3 | 7,10 - 7,79 | 3,29 -6,79 | 0,708 3,63 |
| NEU range | 0,907 0,816 | 1,76 - 1,94 | 0,476 1,46 | 0,347 0,868 |
| LYM range | 5,94 - 7,55 | 4,47 - 5,30 | 1,52 -5,36 | 0,255 -2,23 |
| MONO range | 0,422 0,458 | 0,519 0,725 | 0,247 0,300 | 0,089 0,286 |
| EOS range | 0,009 0,015 | 0,003 -0,044 | 0,005 0,014 | 0,007 0,008 |
| BASO range | 0,044-0,076 | 0,029-0,098 | 0,033-0,057 | 0,008-0,021 |
| RBC range | 8,34 - 8,66 | 6,62 - 8,20 | 6,64 -7,55 | 6,80 -8,63 |
| HGB range | 13,1 - 13,2 | 10,3 - 12 | 10,3 -11,4 | 10,6 -12,3 |
| HCT range | 38,1 - 39,6 | 30,2 - 36,6 | 31,5 -33,4 | 31,1 -38,8 |
| MCV range | 44,4 - 45,6 | 44,6 - 44,6 | 43,7 -44,2 | 44,8 -44,9 |
| MCH range | 15 - 15,1 | 14,6 - 14,6 | 14,7 -15,1 | 14,6 -15,1 |
| MCHC range | 32,9 - 33,5 | 32,7 - 32,7 | 32,6 -33,7 | 32,6 -33,6 |
| RDW range | 15,6 - 16 | 16,1 - 17 | 15,3 -16,9 | 15,8 -16,5 |
| PLT range | 1089 -1091 | 1289 -1508 | 1220 -1282 | 768 - 966 |
| MPV range | 8,71 - 8,87 | 8,63 - 8,75 | 8,32 -8,65 | 8,79 -9,66 |
| WIC range | 8,47 - 11,3 | 7,57 - 13,8 | 4,44 -7,27 | 0,847 3,63 |
| WOC range | 7,82 - 8,13 | 6,58 - 12 | 3,29 -6,46 | 0,708 2,89 |

## Revendications

1. Composé d'origine végétale de type terpène pour son utilisation pour prévenir ou traiter la dégénérescence de tissus associée à la sénescence des cellules endothéliales vasculaires induite par des épisodes inflammatoires répétés.

2. Composé d'origine végétale de type terpène pour son utilisation selon la revendication 1, **caractérisé en ce que** le composé d'origine végétale de type terpène est choisi dans le groupe comprenant l'acétate de géranyl, le géraniol, l'isomenthone et le limonène.

3. Composé d'origine végétale de type terpène pour son utilisation selon la revendication 1 ou 2, **caractérisé en ce que** le composé d'origine végétale de type terpène est isolé et purifié ou préparé par synthèse.

4. Composé d'origine végétale de type terpène pour son utilisation selon l'une quelconque des revendications 1 à 3, pour son utilisation pour prévenir ou traiter l'exportation des métastases de tumeurs par les cellules endothéliales vasculaires sénescentes.

5. Composé d'origine végétale de type terpène pour son utilisation selon l'une quelconque des revendications 1 à 3, pour son utilisation pour traiter les affections dermatologiques et les allergies cutanées liées à la pollution, aux micro abrasions, qui entraînent une surexpression des molécules d'adhérence par l'endothélium vasculaire et une polymérisation de l'actine endothéliale vasculaire.

6. Composé d'origine végétale de type terpène pour son utilisation selon l'une quelconque des revendications 1 à 3, pour son utilisation pour traiter une pathologie inflammatoire chronique ou auto-immune qui entraîne une surexpression des molécules d'adhérence par l'endothélium vasculaire et une polymérisation de l'àctine endothéliale vasculaire.

7. Composé d'origine végétale de type terpène pour son utilisation selon la revendication 6, **caractérisé en ce que** la pathologie inflammatoire chronique ou auto-immune est choisie dans le groupe comprenant la polyarthrite rhumatoïde, l'ostéoarthrite, les spondylarthrites, la goutte, l'arthrose et toutes les autres formes d'arthrites, l'hépatite chronique, la rectocolite hémorragique, la maladie de Crohn, les vasculites, la sclérose en plaques, le psoriasis, les lupus érythémateux et cutané ainsi que les sclérodermies.

8. Composé d'origine végétale de type terpène pour son utilisation selon l'une quelconque des revendications 1 à 3, pour son utilisation pour traiter les maladies dégénératives cérébrales qui sont associées à une surexpression des molécules d'adhérence par l'endothélium vasculaire et une polymérisation de l'actine endothéliale vasculaire.

9. Composé d'origine végétale de type terpène pour son utilisation selon la revendication 8, **caractérisé en ce que** la pathologie dégénérative cérébrale est choisie dans le groupe comprenant la maladie d'Alzheimer, les démences séniles vasculaires ou mixtes, la maladie de Parkinson, la maladie de Huntington,

10. Composé d'origine végétale de type terpène pour son utilisation selon l'une quelconque des revendications 1 à 9, **caractérisé en ce que** le composé est administré par voie topique, orale, entérale, parentérale ou par inhalation,

## Patentansprüche

1. Pflanzliche Zusammensetzung vom Typ Terpen für ihre Verwendung zur Vorbeugung oder Behandlung der Degeneration von Gewebe in Verbindung mit der Seneszenz der vaskulären Endothelzellen durch wiederholte Entzündungsepisoden.

2. Pflanzliche Zusammensetzung vom Typ Terpen für ihre Verwendung nach Anspruch 1, **dadurch gekennzeichnet, dass** die pflanzliche Zusammensetzung vom Typ Terpen aus der Gruppe ausgewählt ist, die das Geranylacetat, das Geraniol, das Isomenthon und das Limonen umfasst.

3. Pflanzliche Zusammensetzung vom Typ Terpen für ihre Verwendung nach Anspruch 1 oder 2, **dadurch gekennzeichnet, dass** die pflanzliche Zusammensetzung vom Typ Terpen isoliert und gereinigt oder synthetisch zubereitet ist.

4. Pflanzliche Zusammensetzung vom Typ Terpen für ihre Verwendung nach einem der Ansprüche 1 bis 3 für ihre Verwendung zur Vorbeugung oder Behandlung des Exports von Tumormetastasen durch seneszente vaskuläre Endothelzellen.

5. Pflanzliche Zusammensetzung vom Typ Terpen für ihre Verwendung nach einem der Ansprüche 1 bis 3 für ihre Verwendung zur Behandlung dermatologischer Erkrankungen und Hautallergien, die mit der Verschmutzung, mit Mikroabrasionen verbunden sind, die zu einer Überexpression der Adhäsionsmoleküle durch das vaskuläre Endothel und eine Polymerisation des vaskulären Endothelaktins führen.

6. Pflanzliche Zusammensetzung vom Typ Terpen für ihre Verwendung nach einem der Ansprüche 1 bis 3 für ihre Verwendung zur Behandlung einer chronischen entzündlichen oder Autoimmunpathologie, die zu einer Überexpression der Adhäsionsmoleküle durch das vaskuläre Endothel und eine Polymerisation des vaskulären Endothelaktins führt.

7. Pflanzliche Zusammensetzung vom Typ Terpen für ihre Verwendung nach Anspruch 6, **dadurch gekennzeichnet, dass** die chronischen entzündliche oder Autoimmunpathologie aus der Gruppe ausgewählt ist, die die rheumatoide Polyarthritis, die Osteoarthritis, die Spondylarthritiden, die Gicht, die Arthrose und alle anderen Arthritisformen, die chronische Hepatitis, die Rektokolitis hämorrhagica, den Morbus Chron, die Vaskulitiden, die Multiple Sklerose, die Psoriasis, den Lupus erythematodes und cutaneus sowie die Sklerodermien umfassen.

8. Pflanzliche Zusammensetzung vom Typ Terpen für ihre Verwendung nach einem der Ansprüche 1 bis 3 für ihre Verwendung zur Behandlung der zerebralen degenerativen Krankheiten, die mit einer Überexpression der Adhäsionsmoleküle durch das vaskuläre Endothel und eine Polymerisation des vaskulären Endothelaktins verbunden sind.

9. Pflanzliche Zusammensetzung vom Typ Terpen für ihre Verwendung nach Anspruch 8, **dadurch gekennzeichnet, dass** die zerebrale degenerative Pathologie aus der Gruppe ausgewählt ist, die die Alzheimerkrankheit, die vaskulären oder gemischten senilen Demenzen, die Parkinsonsche Krankheit, die Huntington-Krankheit umfasst.

10. Pflanzliche Zusammensetzung vom Typ Terpen für ihre Verwendung nach einem der Ansprüche 1 bis 9, **dadurch gekennzeichnet, dass** die Zusammensetzung auf topischem, oralen, parenteralem Weg oder durch inhalation verabreicht wird.

## Claims

1. Plant-origin compound of terpene type for use for preventing or treating tissue degeneration associated to the senescence of vascular endothelial cells induced by repeated inflammatory episodes.

2. Plant-origin compound of terpene type for use according to claim 1, **characterized in that** the plant-origin terpene compound is selected from the group comprising of geranyl acetate, geraniol, isomenthone and limonene.

3. Plant-origin compound of terpene type for use according to claim 1 or 2, **characterized in that** the plant-origin compound of terpene type is isolated and purified or prepared by chemical synthesis.

4. Plant-origin compound of terpene type for use according to any of claims 1 to 3, for use for preventing or treating the export of tumour metastases by the senescent vascular endothelial cells.

5. Plant-origin compound of terpene type for use according to any of claims 1 to 3, for use for treating dermatological diseases and skin allergies related to pollution, to micro abrasions, which cause overexpression of adhesion molecules by the vascular endothelium and polymerization of vascular endothelial actin.

6. Plant-origin compound of terpene type for use according to any of claims 1 to 3, for use for treating a chronic inflammatory disease or autoimmune disease that leads to overexpression of adhesion molecules by the vascular endothelium and polymerization of the vascular endothelial actin.

7. Plant-origin compound of terpene type for use according to claim 6, **characterized in that** the chronic inflammatory disease or autoimmune disease is selected from the group comprising rheumatoid arthritis, osteoarthritis, spondylarthritis, gout, arthrosis and any other form of arthritis, chronic hepatitis, ulcerative colitis, Crohn's disease, vasculitis, multiple sclerosis, psoriasis, systemic and cutaneous lupus erythematosus, and scleroderma.

8. Plant-origin compound of terpene type for use according to any one of claims 1 to 3, for use to treat degenerative brain diseases that are associated with an overexpression of adhesion molecules by the vascular endothelium and polymerization of the vascular endothelial actin.

9. Plant-origin compound of terpene type for use according to claim 8, wherein the degenerative brain disease is selected from the group comprising Alzheimer's disease, vascular or mixed senile dementia, Parkinson's disease, and Huntington's disease.

10. Plant-origin compound of terpene type for use according to any one of claims 1 to 9, **characterized in that** the compound is administered topically, orally, enterally, parenterally or by inhalation.
